# EUROPEAN PATENT APPLICATION

(11) **EP 1 695 723 A2**
(43) Date of publication of application: **30.08.2006**
(21) Application number: 06001396.8
(22) Date of filing: 24.01.2006
(51) Int. Cl.: A61L 15/58

(54) **Pressure sensitive adhesive for surgical drapes**

(30) Priority: 25.02.2005 US 65844
(71) Applicant: Tyco Adhesives LP, Franklyin, MA 02038 (US)
(72) Inventor: Cheney, Grant, Grand Rapids, MI 49525 (US); Cooper, Johnnie, Corona, CA 92881 (US); Lamarre, Gerard, Franklin, MA 02038 (US)
(74) Representative: Hafner, Dieter

(57) **Abstract**

The invention relates to the selection and application of adhesive systems that facilitate bonding to generally non-polar and fibrous hydrocarbon surfaces, and which adhesive also provides physical properties (e.g., viscosity flow) to promote improved mechanical interlocking with the substrate fiber network. The substrate comprises a non-woven barrier fabric to which an adhesive incorporating a tackifier is applied.

## Description

### FIELD OF INVENTION

This invention relates to adhesive-coated drapes, e.g., surgical drapes, which provide adhesion as between the drapes and/or a patient. More specifically, the invention herein relates to the selection and application of adhesive systems that facilitate bonding to generally non-polar and fibrous hydrocarbon surfaces, and which adhesive also provide physical properties (e.g., viscosity flow) to promote improved mechanical interlocking with the substrate fiber network.

### BACKGROUND

Nonwoven barrier fabrics have been developed to impede the passage of bacteria and other contaminants and are used for disposable medical fabrics, such as surgical drapes, disposable gowns and the like. For example, such barrier fabrics can be formed by sandwiching an inner fibrous web of thermoplastic meltblown microfibers between two outer nonwoven webs of substantially continuous thermoplastic spunbonded filaments. The fibrous meltblown web provides a barrier impervious to bacteria or other contaminants in the composite nonwoven fabric. Such composite fabric structures are known generally in the art as spunbond-meltblown-spunbond, or "SMS," fabrics. Examples of such fabrics are described in U.S. Pat. No. 4,041,203 and U.S. Pat. No. 4,863,785.

Other barrier fabrics, for example, U. S. Patent No. 4,695, 334 to Mays, include a multiple layer plastic film that is fused or thermally bonded to at least one layer of conjugate fibers having a low melting sheath and a high melting core. The sheaths of the conjugate fibers are fuse bonded to the plastic film at a temperature below the melt temperature of the cores of the conjugate fibers so that the cores retain their initial fiber-like integrity.

Furthermore, it is sometimes necessary to adhere non-woven barrier fabrics to other non-woven barrier fabrics or to the skin of a patient. Pressure-sensitive adhesive tapes and the like are reportedly used, such as an acrylic pressure sensitive adhesive, in an effort to satisfy ISO 10993 medical product requirements.

While these embodiments certainly contribute to the field of providing a non-woven barrier fabric, a need remains to expand upon and improve upon the above, particularly with respect to the compatibility of the adhesives with different types of non-woven barrier fabrics.

Accordingly, it is one object of the present invention to provide an adhesive that may be utilized more efficiently in conjunction with non-woven barrier fabrics. It is also an object of the present invention to provide an adhesive that may be suitable for skin contact. Furthermore, it is an object of the present invention to provide appropriate bond strength to supply a useful nonwoven barrier fabric for various medical applications.

### SUMMARY

An aspect of the present invention relates to an adhesive formulation for a non-woven substrate comprising a polymer adhesive composition comprising a polymer and tackifier mixture, wherein the tackifier may be present at about 80-250 parts per 100 parts of adhesive.

Another aspect of the present invention relates to an adhesive coated drape comprising a drape comprising a layer of spun bond fiber, having an exposed surface; wherein the spun bond fiber is a polymeric fiber with a solubility parameter **δ**_{**fiber**}; a pressure sensitive adhesive applied to the exposed surface wherein the pressure sensitive adhesive comprises a polymeric resin with a solubility parameter **δ**_{**adhesive**} wherein **δ**_{**fiber**} is within 2-4 units of **δ**_{**adhesive**} as measured in (cal/cm³)^{0.5}; and a tackifying resin mixed with the pressure sensitive adhesive wherein the tackifying resin is present in an amount of about 80-250 parts per 100 parts of rubber.

### DETAILED DESCRIPTION

The drape herein may include any nonwoven material, such as a spun bond polyolefin layer of material. Those of skill in the art will recognize that a spun bond material is reference to the formation of a material in which filaments have been extruded, drawn or placed on a moving screen to form a web. More particularly, where a spunbond substrate is extruded, the nonwoven material may be constructed from relatively small diameter fibers which may be formed by extruding molten thermoplastic material as filaments from a plurality of fme, usually circular capillaries of a spinneret. The diameter of the extruded filaments may then be reduced further if desired.

Spunbond fibers are generally not tacky when they are deposited onto a collecting surface. Spunbond fibers are generally continuous and have average diameters (from a sample of at least 10) larger than 7 microns, more particularly, between about 10 and 25 microns. Further, substrates produced using other nonwoven technologies, such as carding, hydro-entangling and wet-laid processes, may also be used to produce the nonwoven laminate product of this invention. Each nonwoven substrate may preferably have a basis weight in the range of 10-100 gsm.

The drape herein may also include a film layer created by the cast lamination process, which film may be disposed on the spun bond polyolefin layer of material or between layers of the spun bond polyolefin. Those of skill in the art will recognize that the cast lamination process is a reference to the formation of a cast film produced using a conventional extrusion process. Multiple extruders may be used to process and melt different polymers and then specially designed selector plugs and feed-blocks may be used to combine the different polymers into different layers to form a multiple layer structure such as A-B, A-B-A, A-B-C, A-B-B-A, etc. The film may be extruded from a relatively wide die followed by cooling using a chill roll. After production, the cast film may further be embossed. The cast film may also be wound on a roll for storage and/or transportation.

To form the composite nonwoven product, the cast film may be placed upon and laminated to one or more nonwoven substrates on one or both sides of the film. A preferred lamination method may use adhesives. Such adhesive may be preferably applied using spray nozzles. The pattern may be chosen to optimize adhesion and softness or the ability to drape. Some representative examples of commercially available patterns include: Control Weave, Meltblown and UFD (Uniform fiber deposition). The number of holes per inch in the nozzle may be chosen based on the intended end-use.

Other techniques for applying adhesive, include gravure coating, slot coating and powder coating. The adhesive for the layers is preferably applied to have a dry basis weight in the range of 1-10 gsm. If hot melt adhesives are used, the application temperature should not be so high as to damage the cast film. The completed composite nonwoven laminate may then be wound on a roll for storage and/or transportation. The foregoing cast-lamination process may be completed as a one-step process or may be broken into multiple separate steps.

The cast film preferably comprises multiple layers and is typically made from at least two polyolefinic resins, such as polyethylene resins, polypropylene homopolymers/copolymers, low density polyethylene (LDPE), linear low density polyethylene (LLPDE), ethyl vinyl acetate (EVA), ethylene methyl acrylate (EMA), maleic anhydride modified polyethylene (PE), amorphous polypropylene (PP), crystalline PP, random copolymers (RCP) of PP and PE, or blends thereof. Key properties in choosing suitable polymers are Melt Index, density and melting point. For polyethylene resins, the preferable melt index range is from 1-15 MFI. For polypropylene resins the preferable melt flow rate range is from 15-50 MFR. When producing a bi-laminate nonwoven product (i.e., two layers of film) the cast film should preferably have at least two layers having different constituent polymer parts (i. e., A-B). In the bi-laminate, the nonwoven substrate is in contact with and bonded to the A layer and when producing a tri-laminate nonwoven product, the cast film should preferably have at least three layers of which at least two of the layers preferably have different constituent parts (i. e. , A-B-A), where the A layers are in contact with and bonded to the nonwoven substrate.

Other cast film structures, such as A-B-B-A, A-B-C, may be employed depending on the end use application. At least one layer may preferably provide the barrier properties sufficient to prevent blood, viruses and bacteria from passing through; for example, the core layer (B) may comprise LDPE or a blend of LDPE and LLDPE for improved performance.

Preferred cast films have a basis weight in the range of 5-50 gsm and wherein the combined weight of said two outer layers lies between 3% and 90% of the total film weight in a ratio of outer layer to barrier layer to outer layer of from 1.5-97-1. 5 to 45-10-45 by weight.

The combined weight of the two outer layers in a trilaminate preferably lies between 3% and 90% of the total film and the layers of the film do not have to be symmetrical. For example, in an A-B-A film, the ratio could be 3-90-7 by weight.

In preferred embodiments, the nonwoven substrate is a spunbond polypropylene substrate. However, substrates produced using other fibers such as PE, PET (polyester), bi-component fibers PE/PET may also be used.

To adhere the drape to skin or another drape, it has been found that this can now be optimized when an adhesive is employed of the type and characteristics disclosed herein. In general terms, the adhesive is preferably one which provides improved bond strength to the substrate fibrous surface region by selecting an adhesive that is compatible with the fibrous surfaces. By compatible, it is meant that the adhesive is selected such that its intermolecular interactions with the surface of the fiber is complimentary and the surfaces do not oppose each other with respect to the intermolecular forces. For example, in the case of a non-polar polyolefm fiber, it has been found useful to select an adhesive that has related hydrocarbon character and which therefore will not be rejected by the non-polar nature of the fiber. In that regard, one selection criterion is to select an adhesive that provides a solubility parameter (δ) measured in (cal/cm³)^{0.5} that is within about 2-4 units of the substrate fiber, more preferably within 2-3 units, and most preferably 1-2 units, or 0.1-1.0 units, including all incremental values therebetween.

Preferably, for the polyolefin fiber surface network, the adhesive is a styrene block copolymer adhesive. More preferably, the styrene block copolymer may be comprised of an A-B-A copolymer where the A block can be thermoplastic polystyrene endblocks and the B block represents a rubber midblock including but not limited to polyisoprene, polybutadiene, poly (ethylene/butylene) or poly (ethylene/propylene). In one embodiment, the adhesive may be a polystyrene-polyisoprene-polystyrene block copolymer, available from Dexco Polymers, and sold under the trademark VECTOR. Other suppliers of styrenic block copolymers include but are not limited to rubber copolymers available from Kraton sold under the trademark KRATON, from Asahi Kasei sold under the trademark TUFPRENE, from Enichem sold under the trade name EUROPRENE SOL T, from Fina sold under the trade name FINAPRENE, from Firestone sold under the trade name STEREON, from Kuraray sold under the trade name SEPTONE, from Nippon Zeon sold under the trade name QUINTAC, and from TSR sold under the trade name TAIPOL. Such styrene block copolymer may be a multi-arm radial block copolymer or it may be a linear polymer.

Accordingly, it may preferably comprise the structure [PS-PI]nX, wherein PS is polystyrene, PI is polyisoprene, and X is a multifunctional coupling agent employed in the production of the radial or linear block copolymer. The average value of n may range up to about 7, and may be in the range of 3-7, and all incremental values therebetween. In a particularly preferred embodiment, n may have a value of about 4. It is also worth noting that the preferred VECTOR 4230 is a four arm radial block copolymer, with a coupling efficiency of 70%, i.e. 30% diblock. In addition, the VECTOR 4230 provides a melt flow rate of about 14 g/10 minutes, but in the broad context of the present invention, the melt flow rate of the adhesive may be within the range of 10-20 g/10 minutes, and all incremental values there between. In addition, the preferred adhesive may have a tensile strength of about 1500-3000 psi, and all incremental values there between, elongation of between about 800-1200% (for compression molded plaques) and all incremental values there between. Furthermore the preferred adhesive has a Shore A hardness of between about 30-50. The specific gravity may be about 0.9-1.0. In addition, the adhesive preferably resists degradation upon exposure to gamma radiation.

In one particularly preferred embodiment, a tackifier resin may be added into the adhesive system. The tackifier is preferably one that is similarly compatible with the adhesive and which provides relatively low viscosity to improve the flow characteristics so that the adhesive and tackifier may provide a mechanical interlock with the fibers of the nonwoven substrate. Tackifiers may be petroleum based resins and polyterpenes containing only carbon and hydrogen, or they may also contain oxygen as in coumarone-indene resins and rosin esters. Many resin varieties and grades are available, and may tackify the end blocks and the midblocks of the preferred block copolymer adhesive.

The tackifier may therefore be any hydrocarbon resin, including aliphatic and aromatic based resins, as well as aromatic modified aliphatic hydrocarbon resins, that are preferably compatible with the adhesive and/or which preferably provide a Brookfield melt viscosity at 140° C of about 1500-4500 centipoise, and all incremental values therebetween. Also, preferably, the tackifier has a weight average molecular weight (Mw) of between about 1250 to 1450, a number average molecular weight (Mn) of about 500 - 1000 and a z average molecular weight (Mz) of about 1500-3000, and all incremental values there between for such molecular weight variables (Mw, Mn and/or Mz). Preferably, the tackifier may have a Tg of between about 35-55°C, and all incremental values there between. More preferably the Tg is about 45°C. Preferably, the tackifer may be obtained from Exxon Mobil and is sold under the trade name Escorez®. Other vendors of tackifying resins include, but are not limited to, Hercules sold under the trade names FORAL and PENTALYN, Arizona Chemical, sold under the trade names SYLVATAC and ZONESTER, Eastman Chemical sold under the trade name EASTOTAC, Goodyear sold under the grade name WINGTACK, Neville Chemical sold under the trade name KUMAR, and Nippon Zeon sold under the trade name QUINTONE.

In one embodiment, an additional tackifier resin may be added to the adhesive system. Preferably, the additional tackifier may be an ester or phenolic resin. More preferably, the tackifier may be a thermoplastic, unmodified alkylphenolic resin.

In further embodiment, additives may be also be added to the adhesive. Additives that may be added include antioxidants and oils. Preferably, the anti-oxidant is a liquid antioxidant. The anti-oxidant may also act as a thermal stabilization system. Various chemicals can provide antioxidant protection; including but not limited to secondary amines, diamines and their derivatives, compounds of quinoline, dithiocarbamates, alkyl phenols and phosphoric acid esters. Preferably, the antioxidant may be obtained from Mayzo, Norcross, Georgia and is sold under the trade name BNX®. Preferably, the oils included are naphthenic process oils but they may also be aromatic or parafinnic oils. Preferably, the oils may be obtained from Ergon, Jackson, MS, and sold under the trade name Hyprene.

In a preferred embodiment the tackifier may be present at amounts between 80 to 250 parts per 100 parts of adhesive, and all intervals there between. More preferably, the tackifier may be present at amounts between 150 to 200 parts per 100 parts adhesive. Most preferably, the tackifier may be present at amounts between 170 to 190 parts per 100 parts of adhesive.

In another optional embodiment, the tackifier may be present at amounts between 170 to 190 parts per 100 parts of adhesive and the additional tackifier is present between about 1 and 50 parts per 100 parts of adhesive and all incremental values there between. More preferably, the additional tackifier may be present at about 10-25 parts to 100 parts of adhesive.

Furthemore, in another embodiment, the antioxidant may be present at levels between 1-10 parts per 100 parts of adhesive. Also preferably, the oils may be present at levels between 25 and 40 parts per 100 parts of adhesive.

### EXAMPLES

The following examples are offered to aid in understanding the present invention and are not to be construed as limiting the scope thereof.

### Example 1

Two adhesive blends were tested to determine peel strength of the adhesives when applied to a polyolefin nonwoven substrate such as would occur if the adhesive was being used on the nonwoven substrate, such as adhering one surgical drape upon another. The tests were performed per the Pressure Sensitive Tape Counsil's PSTC-101 International Test Method. The first adhesive blend was a medical-grade tackified acrylic adhesive. The second adhesive blend was an embodiment of the present invention containing approximately 100 parts of styrenic block copolymer rubber and approximately 180 parts of tackifier. Two different bond pressures were used. The results of the peel strength test are illustrated in Table 1.

**Table 1. Peel Strength to Tiburon, in lbf/in**

| Adhesive | 0.9 pounds of applied pressure to form the bond | 4.5 pounds of applied pressure to form the bond |
|---|---|---|
| tackified acrylic | 14 | 12 |
| present adhesive and tackifier | 25 | 45 |

### Example 2

Two adhesive blends were tested to determine the shear strength of the adhesives when applied to TIBURON (a polyolefm nonwoven substrate containing film layers). The first adhesive blend was a medical-grade tackified acrylic adhesive. The second adhesive blend was an embodiment of the present invention containing approximately 100 parts of adhesive and approximately 180 parts of tackifier. The adhesive was applied to Tiburon (absorbent side out) which was applied to a double sided tape which was applied to a stainless steel panel and then tested in accordance with the Pressure Sensitive Tape Council's PSTC-107 International Standard for Shear Adhesion of Pressure Sensitive Tapes using a 1 inch by 1 inch bond area and 150 grams of weight.

Prior to testing, the adhesives were exposed to ethylene oxide to determine the comparative ability of the adhesives to withstand sterilization. Single and double exposure cycles were examined. The results of the shear tests are summarized in Table 2, data is presented in minutes. Shear strength results are similar for both the absorbent and nonabsorbent sides of the Tiburon.

**Table 2. Shear Test, in minutes**

| Adhesive | Unsterilized | 1 ethylene oxide cycle | 2 ethylene oxide cycles (*absorbent*) |
|---|---|---|---|
| tackified acrylic | 85 | 105 | 65 |
| present adhesive | 40 | 240 | 120 |

### Example 3

The peel strength of two adhesive blends were tested. when applied to a polyolefin nonwoven substrate. The first adhesive blend was a medical-grade tackified acrylic adhesive. The second adhesive blend was an embodiment of the present invention containing approximately 100 parts of adhesive and approximately 180 parts of tackifier. The tests were performed per the Pressure Sensistive Tape Council's PSTC-101 International Test Method.

Prior to testing, the adhesives were also exposed to ethylene oxide to determine the ability of the systems to withstand sterilization. Single and double exposure cycles were examined. The results of the peel strength tests are summarized in Table 2, data is presented in Ib_{f}/in.

**Table 3. Peel Strength, in lb_{f}/in**

| Adhesive | Unsterilized | 1 ethylene oxide cycle | 2 ethylene oxide cycles (*absorbent*) | 2 ethylene oxide cycles (*nonabsorbent*) |
|---|---|---|---|---|
| tackified acrylic | 2.2 | 2.1 | 2.5 | 2.0 |
| present adhesive | 2.7 | 8.3 | 6.2 | 8.5 |
| tackified acrylic | 2.4 | 2.3 | 2.8 | 2.4 |
| present adhesive | 2.6 | 8.4 | 7.0 | 7.8 |
| tackified acrylic | clean removal | clean removal | clean removal | clean removal |
| present adhesive | 2.5 clean removal | 8.4 ≥ 50% cohesive failure | 7.5 ≥ 50% cohesive failure | 7.5 ≥ 50% cohesive failure |

The foregoing description is provided to illustrate and explain the present invention. However, the description hereinabove should not be considered to limit the scope of the invention set forth in the claims appended here to.

## Claims

1. An adhesive formulation for a non-woven substrate comprising:
a polymer adhesive composition comprising a polymer and tackifier mixture, wherein said tackifier is present at about 80-250 parts per 100 parts of adhesive.

2. The adhesive formulation of claim 1 wherein said tackifier is present at about 150-200 parts per 100 parts of adhesive.

3. The adhesive formulation of claim 1 wherein said polymer has a melt flow index of about 10-20 g/10 min.

4. The adhesive formulation of claim 1 wherein said tackifier has a Brookfield melt viscosity of about 1500-4500 centipoise at 140° C.

5. The adhesive formulation of claim 1 wherein said polymer is a styrene block copolymer.

6. The adhesive formulation of claim 5 wherein said styrene block copolymer is a poly(styrene-isoprene) block copolymer and said poly(styrene-isoprene) based block copolymer comprises the structure [PS-PI]nX, where PS is polystyrene, PI is polyisoprene, and X is a multifunctional coupling agent and n is an average value between 3 and 7.

7. The adhesive formulation of claim 6 wherein said styrene-isoprene block copolymer is a multi-arm radial copolymer.

8. The adhesive formulation of claim 1 wherein said tackifier is a hydrocarbon resin.

9. The adhesive formulation of claim 1 wherein said tackifier is selected from the group consisting of aliphatic based resins, aromatic based resins, aromatic modified aliphatic based resins and mixtures thereof.

10. The adhesive formulation of claim 1 wherein said polymer provides mechanical interlocking to a fibrous substrate.

11. An adhesive coated drape comprising:
a drape comprising a layer of spun bond fiber, having an exposed surface; wherein said spun bond fiber is a polymeric fiber with a solubility parameter **δ**_{**fiber**}**,**
a pressure sensitive adhesive applied to said exposed surface wherein the pressure sensitive adhesive comprises a polymeric resin with a solubility parameter **δ**_{**adhesive**} wherein **δ**_{**fiber**} is within 2-4 units of **δ**_{**adhesive**} as measured in (cal/cm³)^{0.5}; and
a tackifying resin mixed with said pressure sensitive adhesive wherein said tackifying resin is present in an amount of about 80-250 parts per 100 parts of adhesive.

12. The drape of claim 11 wherein said polymeric fiber is a polyolefin fiber.

13. The drape of claim 11 wherein said pressure sensitive adhesive is a styrene block copolymer.

14. The drape of claim 11 wherein said pressure sensitive adhesive is a poly(styrene-isoprene) based block copolymer and said poly(styrene-isoprene) based block copolymer comprises the structure [PS-PI]nX, where PS is polystyrene, PI is polyisoprene, and X is a multifunctional coupling agent and n is an average value between 3 and 7.

15. The drape of claim 11 wherein said tackifier is a hydrocarbon resin.

16. The drape of claim 11 wherein said tackifying resin is selected from the group consisting of aliphatic based resins, aromatic based resins, aromatic modified aliphatic based resins and mixtures thereof.

17. The drape of claim 11 wherein said tackifying resin is present between 80-250 parts per 100 parts of pressure sensitive adhesive.

18. The drape of claim 11 wherein said tackifying is present between 150-200 parts per 100 parts of pressure sensitive adhesive.

19. The drape of claim 11 further comprising a film layer bonded to said spun bond fiber.

20. The drape of claim 11 wherein said tackifying resin mixed with said pressure sensitive adhesive provides a mechanical bond to said spun bond fiber.
